# EUROPÄISCHE PATENTSCHRIFT

(11) **EP 2 288 584 B1**
(45) Veröffentlichungstag und Bekanntmachung des Hinweises auf die Patenterteilung: **26.06.2013**
(21) Anmeldenummer: 09757367.9
(22) Anmeldetag: 28.04.2009
(51) Int. Cl.: C07C 7/173, C07C 45/50

(54) **VERFAHREN ZUR ABTRENNUNG VON 1-BUTEN AUS C4-HALTIGEN KOHLENWASSERSTOFFSTRÖMEN DURCH HYDROFORMYLIERUNG**
METHOD FOR SEPARATING 1-BUTENE FROM C4-CONTAINING HYDROCARBON STREAMS BY HYDROFORMYLATION
PROCEDE POUR SEPARER, PAR HYDROFORMYLATION, DU 1-BUTENE DE FLUX D'HYDROCARBURES EN C4

(30) Priorität: 03.06.2008 DE 102008002188
(43) Veröffentlichungstag der Anmeldung: 02.03.2011
(60) Teilanmeldung: 12195793.0
(73) Patentinhaber: Evonik Oxeno GmbH, 45772 Marl (DE)
(72) Erfinder: KREIDLER, Burkard, 45657 Recklinghausen (DE); WIESE, Klaus-Diether, 45721 Haltern am See (DE); HESS, Dieter, 45770 Marl (DE); SELENT, Detlef, 18059 Rostock (DE); BÖRNER, Armin, 18059 Rostock (DE)
(86) Internationale Anmeldenummer: PCT/EP2009/055121
(87) Internationale Veröffentlichungsnummer: WO 2009/146984

(56) Entgegenhaltungen:
- EP-A- 0 016 286
- EP-A- 1 312 598
- WO-A-2008/124468
- DE-A1-102006 058 682
- US-A- 4 668 651
- US-A1- 2002 111 487
- US-A1- 2004 138 508

## Beschreibung

Die vorliegende Erfindung betrifft die selektive Hydroformylierung von 1-Buten zu Valeraldehyd aus C₄-haltigen Kohlenwasserstoffströmen, die sowohl 1-Buten und Isobuten enthalten.

In technischen Strömen liegt Isobuten häufig zusammen mit gesättigten und ungesättigten C₄-Kohlenwasserstoffen vor. Aus diesen Gemischen kann Isobuten wegen der geringen Siedepunktsdifferenz bzw. des sehr geringen Trennfaktors zwischen Isobuten und 1-Buten durch Destillation nicht wirtschaftlich abgetrennt werden. Daher wird Isobuten aus technischen Kohlenwasserstoffgemischen üblicherweise dadurch gewonnen, dass Isobuten zu einem Derivat umgesetzt wird, das sich leicht vom übrig gebliebenen Kohlenwasserstoffgemisch abtrennen lässt, und dass das isolierte Derivat zu Isobuten und Derivatisierungsmittel zurückgespalten wird.

Isobuten ist Ausgangsstoff für die Herstellung einer Vielzahl von Produkten, z. B. für die Herstellung von Butylkautschuk, Polyisobutylen, Isobuten-Oligomeren, verzweigten C₅-Aldehyden, C₅-Carbonsäuren, C₅-Alkoholen und C₅-Olefinen. Weiterhin wird es als Alkylierungsmittel, insbesondere zur Synthese von tert.-Butylaromaten, und als Zwischenprodukt für die Erzeugung von Peroxiden eingesetzt. Darüber hinaus kann Isobuten als Vorstufe für die Herstellung von Methacrylsäure und deren Estern verwendet werden.

Üblicherweise wird Isobuten aus C₄-Schnitten, beispielsweise der C₄-Fraktion eines Steamcrackers, wie folgt abgetrennt:

Nach Entfernung des größten Teils der mehrfach ungesättigten Kohlenwasserstoffe, hauptsächlich Butadien, durch Extraktion(-sdestillation) oder Selektivhydrierung (SHP) zu linearen Butenen, wird das verbleibende Gemisch (Raffinat I oder selektiv hydriertes Crack-C₄) mit Alkohol oder Wasser umgesetzt. Bei der Verwendung von Methanol entsteht aus Isobuten Methyl-tert.-butylether (MTBE), bei Ethanol Ethyl-tert.-butylether (ETBE) und bei Einsatz von Wasser tert.-Butanol (TBA). Nach erfolgter Derivatisierung und Abtrennung können alle drei Produkte in Umkehrung ihrer Bildung zu Isobuten gespalten werden. Für die Isolierung von Isobuten sind neben verschiedenen Trennoperationen demnach mindestens zwei Reaktionsstufen erforderlich.

Alternativ dazu kann Isobuten aus einem C₄-Kohlenwasserstoffstrom, der typischerweise weniger als 1 Massen-% Butadien enthält (C₄-Strom aus Fluid Catalytic Cracking Prozessen, Raffinat I oder selektiv hydriertes Crack-C₄), auf folgende Weise abgetrennt werden:

Das Ausgangsgemisch wird hydriert und isomerisiert, d. h., es wird (noch) vorhandenes Butadien bis zu einem Restgehalt von unter 5 Massen-ppm selektiv hydriert und gleichzeitig 1-Buten zu 2-Butenen isomerisiert. Die Gleichgewichtslage zwischen 1-Buten und den isomeren 2-Butenen liegt z. B. bei 80 °C bei einem Verhältnis von 1 : 17.

Wird die Isomerisierung in einer Reaktivdestillation durchgeführt, kann ein praktisch an 1-Buten freies Kopfprodukt abgetrennt werden, aus dem reines Isobuten weiter aufgearbeitet werden kann. Als Sumpfprodukt wird ein Isobuten-freies Gemisch an 2-Butenen erhalten. Nachteilig ist jedoch, dass das Isobuten eine geringere Reinheit aufweist als das auf dem Weg der Derivatisierung hergestellte Produkt. Weiterhin wird 1-Buten in die reaktionsträgeren wie auch wirtschaftlich unattraktiveren 2-Butene umgewandelt.

Eine weitere Alternative besteht darin, die höhere Reaktivität von 1-Buten gegenüber Isobuten in der Hydroformylierung auszunutzen, um selektiv 1-Buten durch Hydroformylierung aus dem Gemisch zu entfernen. Allerdings sind die Reaktivitätsunterschiede von 1-Buten und Isobuten im Regelfall zu gering, um kommerziell interessante Selektivitäten zu erzielen. In EP 0 016 286 wird ein Verfahren vorgestellt, mit welchem 1-Buten in Gegenwart von Isobuten selektiv zu Valeraldehyd umgesetzt werden kann. Im dort beschriebenen Verfahren wird jedoch ein mindestens 100facher Überschuss an Phosphinligand benötigt, um die gewünschten Selektivitäten zu erzielen, was den Prozess wiederum unattraktiv macht.

Auf dasselbe Verfahren greift die internationale Anmeldung WO 2005/028404 in ihren Beispielen zurück. Dort wird auch, ohne experimentelle Details zu nennen, behauptet, mit dem dort beschriebenen Liganden A bei 65 % 1-Butenumsatz maximal 5 % Isobutenumsatz zu erzielen.

Ein 65%iger 1-Butenumsatz kann jedoch bei weitem nicht als ausreichend erachtet werden, um das technische Problem der Trennung von 1-Buten und Isobuten zufrieden stellend lösen zu können. Eine technische Lösung sollte den 1-Butengehalt weit unterhalb des thermodynamischen Gleichgewichtes zwischen 1-Buten und den isomeren 2-Buten erniedrigen, um anschließend von 1-Buten befreites Isobuten abdestillieren zu können. Als Minimalanforderung kann ein 1-Butenumsatz von 95 % bei maximal 5 % Isobutenumsatz angesehen werden. Bevorzugt ist ein 1-Butenumsatz von mindestens 99 % bei einem Isobutenumsatz von maximal 5 %.

Es bestand daher die technische Aufgabe, ein Verfahren zu entwickeln, mit dem aus einem C₄-haltigen Kohlenwasserstoffgemisch, das Isobuten und 1-Buten enthält sowie 2-Butene enthalten kann, selektiv 1-Buten hydroformyliert, ohne dass Isobuten in gleicher Weise mitreagiert. Idealerweise werden optional enthaltene 2-Butene in möglichst geringem Ausmaße hydroformyliert oder isomerisiert, so dass die Endkonzentration an 1-Buten erheblich unterhalb der Konzentration an 1-Buten im thermodynamischen Gleichgewicht zwischen 1-Buten und 2-Butenen ist. Als Minimalanforderung kann ein 1-Butenumsatz von 95 % bei maximal 5 % Isobutenumsatz angesehen werden. Dabei soll 1-Buten mit einer n/iso - Selektivität von mindestens 97 % am terminalen Kohlenstoffatom zu n-Valeraldehyd hydroformyliert werden. Das Verfahren soll mit erheblich geringeren Ligandenüberschüssen auskommen als EP 0 016 286.

Diese Aufgabe wurde gelöst durch ein Verfahren zur Abtrennung von 1-Buten aus C₄-haltigen Kohlenwasserstoffgemischen, enthaltend Isobuten und 1-Buten, durch Hydroformylierung, wobei das verwendete Katalysatorsystem aus einem der Übergangsmetalle der Gruppen 8 bis 10, bevorzugt Rhodium, und einem Bisphosphitliganden der folgenden Formeln I-1, bis I-3 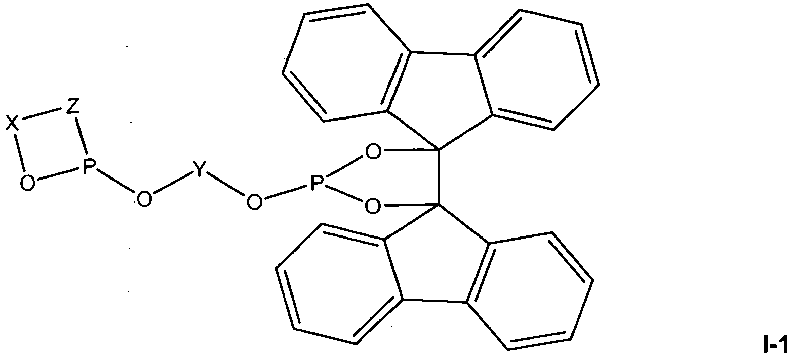 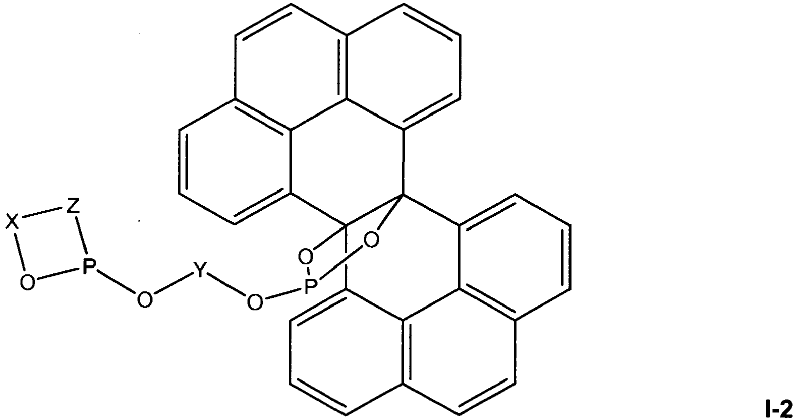 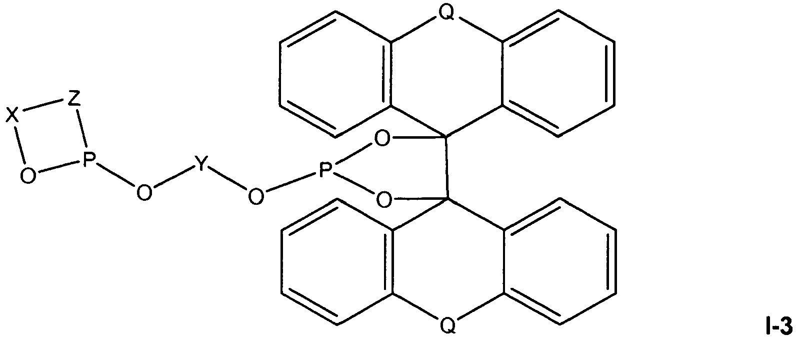 mit X = zweiwertiger substituierter oder unsubstituierter Bisalkylen- oder Bisarylenrest, der ein oder mehrere Heteroatom(e) enthält, Y = zweiwertiger substituierter oder unsubstituierter Bisarylen- oder Bisalkylenrest, der ein oder mehrere Heteroatom(e) enthält, Z = Sauerstoff oder NR⁹ und R⁹ = Wasserstoff oder substituierter oder unsubstituierter Alkyl- oder Arylrest, der ein oder mehrere Heteroatom(e) enthält,
wobei Q z. B. gleich oder verschieden CH₂, CR⁹R¹⁰, CHR⁹, O, NH oder NR⁹ sein kann, wobei R⁹ und R¹⁰ gleich oder unterschiedlich sein können und R⁹ und R¹⁰ = Wasserstoff oder substituierter oder unsubstituierter Alkyl- oder Arylrest, der ein oder mehrere Heteroatom(e) enthält
besteht,
wobei der Bisphosphitligand der oben angegebenen Formel I-1 bis I-3 in einem Überschuss eines molaren Verhältnis von 100 : 1 bis 1 : 1 zum Übergangsmetall eingesetzt wird, und dass bei einem 1-Butenumsatz von über 95 % weniger als 5 % des vorliegenden Isobutens umgesetzt werden.

Es wurde nun gefunden, dass aus einem C₄-haltigen Kohlenwasserstoffgemisch, enthaltend Isobuten und 1-Buten, das 1-Buten durch Hydroformylierung bei Temperaturen unter 120 °C, vorzugsweise unter 100 °C zu C₅-Aldehyden aus dem Kohlenwasserstoffgemisch zu über 99 % umgesetzt werden kann, wobei der Umsatz des vorliegenden Isobuten weniger als 5 % beträgt, wenn ein Katalysatorsystem, bestehend aus Rhodium und einem Bisphosphit, beschrieben in DE 10 2006 058682, eingesetzt wird und die Reaktionstemperatur unter 100 °C liegt. Anschließend wird das Hydroformylierungsgemisch bevorzugt in eine C₅-Aldehydfraktion, reines Isobuten, in eine Katalysatorfraktion und in eine Fraktion mit den übrigen Kohlenwasserstoffen aufgetrennt, wobei die C₅-Aldehydfraktion mit einer n/iso-Selektivität von mehr als 97 % aus Valeraldehyd besteht.

Das erfindungsgemäße Verfahren weist folgende Vorteile auf: Mit nur einer chemischen Umsetzung und einer destillativen Aufarbeitung werden aus einem C₄-Kohlenwasserstoffgemisch mit Isobuten und 1-Buten zwei begehrte Zwischenprodukte, nämlich Isobuten und ein C₅-Aldehydgemisch mit hohem Anteil an Valeraldehyd gewonnen. Darüber hinaus kann ein eventuell anfallendes Kohlenwasserstoffgemisch mit den isomeren 2-Butenen für weitere Umsetzungen, beispielsweise für die Herstellung von Oligomeren oder C₅-Aldehydeh, genutzt werden.

Einsatzstoffe für das erfindungsgemäße Verfahren sind beispielsweise Leichtbenzinfraktionen aus Raffinerien, C₄-Fraktionen aus Spaltanlagen (beispielsweise Steamcrackern, Hydrocrackern, Katcrackern), Gemische aus Fischer-Tropsch-Synthesen, Gemische, entstanden durch Metathese von Olefinen, Gemische, die durch Dehydrierung gesättigter Kohlenwasserstoffe gewonnen werden sowie Gemische, die aus Methanol (oder andere Oxygenate) to Olefin (MTO) - Prozessen entstehen. Diese Techniken sind in der Fachliteratur beschrieben. (H. J. Arpe, Industrielle Organische Chemie, Wiley-VCH, 6. Auflage, 2007, Seiten 9 - 12, 23, 36, 93 - 97, 120).

Bevorzugte Einsatzstoffe von den oben genannten sind C₄-Fraktionen aus Steamcrackern, die primär zur Produktion von Ethen und Propen betrieben werden und in denen als Rohstoffe beispielsweise Raffineriegase, Naphtha, Gasöl, LPG (liquified petroleum gas) und NGL (natural gas liquid) eingesetzt werden, C₄-Fraktionen aus Katcrackern oder Produkte aus Butandehydrieranlagen. Die als Nebenprodukte anfallenden C₄-Schnitte enthalten je nach Verfahren unterschiedliche Mengenanteile an 1,3-Butadien, 1-Buten, Z-2-Buten, E-2-Buten, Isobuten, n-Butan und Isobutan.

Für das erfindungsgemäße Verfahren ist es vorteilhaft, mehrfach ungesättigte Kohlenwasserstoffe, wie 1,3-Butadien, aus dem Einsatzgemisch zu entfernen. Dies kann nach bekannten Verfahren, beispielsweise durch Extraktion, Extraktivdestillation oder Komplexbildung erfolgen (vgl. H. J. Arpe, Industrielle Organische Chemie, Wiley-VCH, 6. Auflage, 2007, Seiten 118 - 119).

Eine Alternative zur Abtrennung der mehrfach ungesättigten Kohlenwasserstoffe ist eine selektive chemische Umsetzung. So kann beispielsweise 1,3-Butadien selektiv zu linearen Butenen hydriert werden, wie z. B. beschrieben in EP 0 523 482. Auch durch selektive Umsetzungen des 1,3-Butadiens, zum Beispiel Dimerisierung zum Cyclooctadien, Trimerisierung zum Cyclododecatrien, Polymerisations- oder Telomerisationsreaktionen, kann das 1,3-Butadien zumindest teilweise entfernt werden. Wurde ein Crack-C₄-Schnitt als Rohstoff eingesetzt, bleibt in allen Fällen ein Kohlenwasserstoffgemisch (z. B. Raffinat I oder hydriertes Crack-C₄ (HCC₄)) zurück, das hauptsächlich die gesättigten Kohlenwasserstoffe, n-Butan und Isobutan, und die Olefine, Isobuten, 1-Buten und 2-Butene, enthält.

Typische Zusammensetzungen von C₄-Schnitten, aus denen der größte Teil der mehrfach ungesättigten Kohlenwasserstoffe entfernt worden sind und die im erfindungsgemäßen Verfahren eingesetzt werden können, sind in der folgenden Tabelle 1 aufgelistet.

**Tabelle 1**

| | Steamcracker | | Steamcracker | | Katcracker | |
|---|---|---|---|---|---|---|
| Komponente | HCC₄ | HCC₄ /SHP | Raff. I | Raff. I / SHP | CC₄ | CC₄ / SHP |
| Isobutan [Massen-%] | 1 - 4.5 | 1 - 4.5 | 1.5-8 | 1.5-8 | 37 | 37 |
| n-Butan [Massen-%] | 5-8 | 5-8 | 6-15 | 6-15 | 13 | 13 |
| E-2-Buten [Massen-%] | 18-21 | 18-21 | 7-10 | 7-10 | 12 | 12 |
| 1-Buten [Massen-%] | 35-45 | 35 - 45 | 15-35 | 15-35 | 12 | 12 |
| Isobuten [Massen-%] | 22 - 28 | 22 - 28 | 33-50 | 33-50 | 15 | 15 |
| Z-2-Buten [Massen-%] | 5-9 | 5-9 | 4-8 | 4-8 | 11 | 11 |
| 1,3-Butadien [Massen-ppm] | 500 - 8000 | 0 - 50 | 50 - 8000 | 0 - 50 | < 10000 | 0 - 50 |

### Erläuterung

- HCC₄: typisch für eine C₄ Mischung, die aus dem Crack-C₄ eines Steamcrackers (High Severity) nach der Hydrierung des 1,3-Butadiens ohne zusätzliche Moderation des Katalysators erhalten wird.
- HCC₄ / SHP: Zusammensetzung HCC₄, bei dem Reste an 1,3-Butadien in einer SHP weiter reduziert wurden.
- Raff. I ((Raffinat I): typisch für eine C₄ Mischung, die aus dem Crack-C₄ eines Steamcrackers (High Severity) nach der Abtrennung des 1,3-Butadiens, beispielsweise durch eine NMP-Extraktivrektifikation, erhalten wird.
- Raff. I / SHP: Zusammensetzung Raff. I, bei dem Reste an 1,3-Butadien in einer SHP weiter reduziert wurden.
- CC₄: typische Zusammensetzung eines Crack-C₄, das aus einem Katcracker erhalten wird.
- CC₄ / SHP: Zusammensetzung CC₄, bei dem Reste an 1,3-Butadien in einer SHP weiter reduziert wurden.

Die Hydroformylierung des Einsatz-Kohlenwasserstoffgemisches, enthaltend Isobuten und 1-Buten, erfolgt unter Bedingungen, bei denen das im Einsatzgemisch vorliegende 1-Buten zu über 95 % und das im Einsatzgemisch vorhandene Isobuten zu weniger als 5 % umgesetzt werden. Bevorzugt wird das im Einsatzgemisch vorliegende 1-Buten zu über 99 % und das im Einsatzgemisch vorhandene Isobuten zu weniger als 5 % umgesetzt. Besonders bevorzugt wird das 1-Buten mit einer n/iso - Selektivität von mehr als 97 % hydroformyliert.

Im Bisphosphit kann der Rest X auch ein Rest Xa sein,

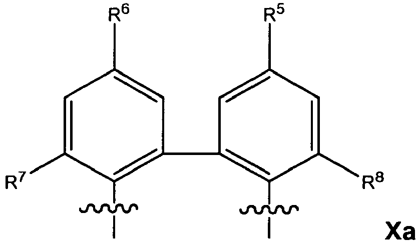

Die Reste R⁵, R⁶, R⁷, R⁸ können unabhängig voneinander substituierte oder unsubstituierte aliphatische, alicyclische, aromatische, heteroaromatische, gemischt aliphatisch-alicyclische, gemischt aliphatisch-aromatische, heterocyclische, gemischt aliphatisch-heterocyclische Kohlenwasserstoffreste mit 1 bis 50 Kohlenstoffatomen oder H, F, Cl, Br, I, -CF₃, -(CH₂)ᵢ(CF₂)ⱼCF₃ mit i = 0 - 9 und j = 0 - 9, -SiR²¹₃, - Si(OR²¹)₃, -SiR²¹(OR²¹)₂, -SiR²¹₂OR²¹, -OSiR²¹₃, -OSi(OR²¹)₃, -OSiR²¹(OR²¹)₂, - OSiR²¹₂OR²¹, -OR¹⁹, -COR¹⁹, -CO₂R¹⁹, -CO₂M, -SO₂R¹⁹, -SOR¹⁹, -SO₃R¹⁹, -SO₃M, -SO₂NR¹⁹R²⁰, -NR¹⁹R²⁰, oder -N=CR¹⁹R²⁰, sein, wobei R¹⁹, R²⁰ und R²¹ unabhängig voneinander aus H, einwertigen substituierten oder unsubstituierten aliphatischen und aromatischen Kohlenwasserstoffresten mit 1 bis 25 Kohlenstoffatomen ausgewählt sind, wobei jedoch R²¹ = H ausgeschlossen ist und M ein Alkalimetall-, Erdalkalimetall-, Ammonium- oder Phosphoniumion ist.

Substituiert können die Reste R⁵, R⁶, R⁷, R⁸ z. B. sein mit einem oder mehreren Resten, ausgewählt aus aliphatischen, alicyclischen, aromatischen, heteroaromatischen, gemischt aliphatisch-alicyclischen, gemischt aliphatischaromatischen, heterocyclischen, gemischt aliphatisch-heterocyclischen Kohlenwasserstoffresten mit 1 bis 50 Kohlenstoffatomen, F, Cl, Br, I, -CF₃, -(CH₂)ᵢ(CF₂)ⱼCF₃ mit i = 0 - 9 und j = 0 - 9, -SiR²¹₃, -Si(OR²¹)₃, -SiR²¹(OR²¹)₂, - SiR²¹₂OR²¹, -OSiR²¹₃, -OSi(OR²¹)₃, -OSiR²¹(OR²¹)₂, -OSiR²¹₂OR²¹, -OR¹⁹, -COR¹⁹, -CO₂R¹⁹, -CO₂M, -SO₂R¹⁹, -SOR¹⁹, -SO₃R¹⁹, -SO₃M, -SO₂NR¹⁹R²⁰, -NR¹⁹R²⁰, oder -N=CR¹⁹R²⁰, wobei R¹⁹, R²⁰, R²¹ und M die oben genannte Bedeutung aufweisen können. Beim Rest Xa stehen die Reste R⁵ bis R⁸ bevorzugt für Wasserstoff, Alkoxygruppen, insbesondere Methoxygruppen oder tert.-Butyl-Gruppen. Bevorzugt sind die Reste R⁵ und R⁶ und die Reste R⁷ und R⁸ jeweils paarweise gleich. Besonders bevorzugt sind die Reste R⁵ und R⁶ Methoxygruppen und/oder die Reste R⁷ und R⁸ tert.-Butyl-Gruppen.

Im Bisphosphit ist das X bevorzugt ein mit Resten R^{1'}, R^{2'}, R^{3'} und R^{4'} substituierter Ethylenrest, wobei die Reste R^{1'}, R^{2'}, R^{3'} und R^{4'} gleiche oder verschiedene, substituierte oder unsubstituierte, verknüpfte, unverknüpfte oder kondensierte Aryl - oder Heteroarylreste sein können. Mögliche Substituenten für die Reste R^{1'} bis R^{4'} sind die für die Reste R¹ bis R⁴ genannten Substituenten. Besonders bevorzugte Bisphosphite sind solche, die symmetrisch sind, d. h. solche, bei denen X ein mit den Resten R^{1'}, R^{2'}, R^{3'} und R^{4'} substituierter Ethylenrest ist, wobei die Reste R¹ und R^{1'}, R² und R^{2'}, R³ und R^{3'} und R⁴ und R^{4'} jeweils gleich sind.

Der zweiwertige Rest Y im erfindungsgemäßen Bisphosphit kann vorzugsweise ein substituierter oder unsubstituierter Bisphenylrest oder Bisnaphthylrest sein. Mögliche Substituenten können die oben genannten Substituenten sein. Bevorzugt ist der Rest Y ausgewählt aus den Bisphenoxyresten der Formeln **IIa** bis **IId** 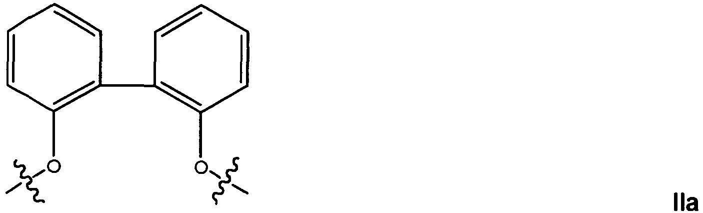 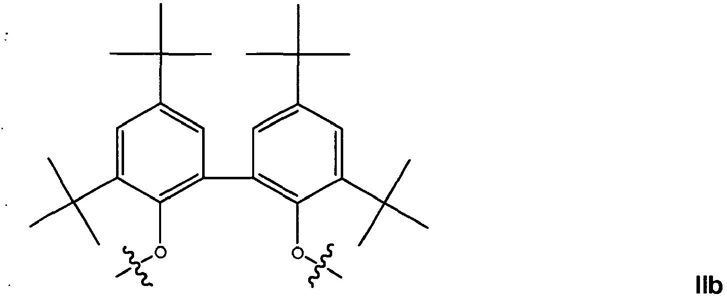 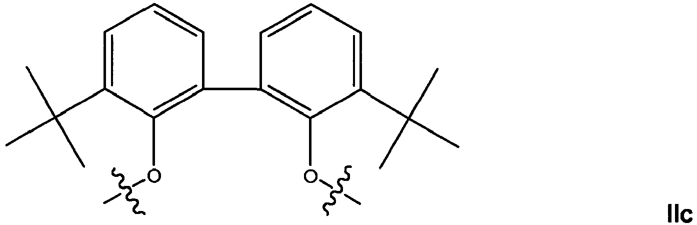 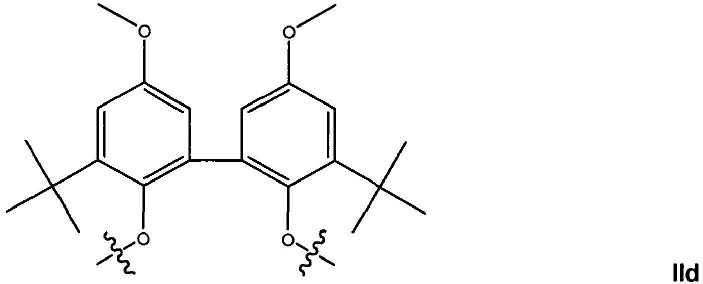 oder Bisnaphthoxyresten der Formel **III** 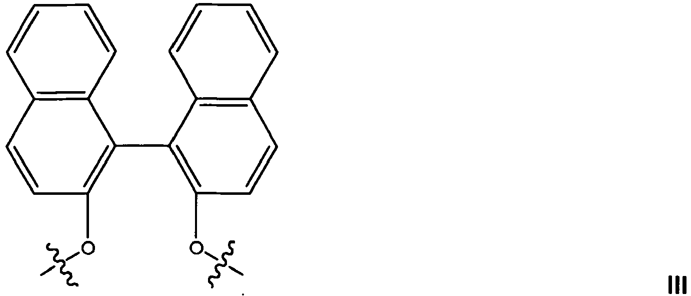 die in racemischer, atropisomerenangereicherter oder atropisomerenreiner Form vorliegen können.

Besonders bevorzugte erfindungsgemäße Bisphosphite sind die Bisphosphite der nachfolgenden Formeln **Ia** bis **Ic,** wobei **Ic** als Racemat oder in atropisomerenangereicherter oder atropisomerenreiner Form dargestellt und eingesetzt werden kann.

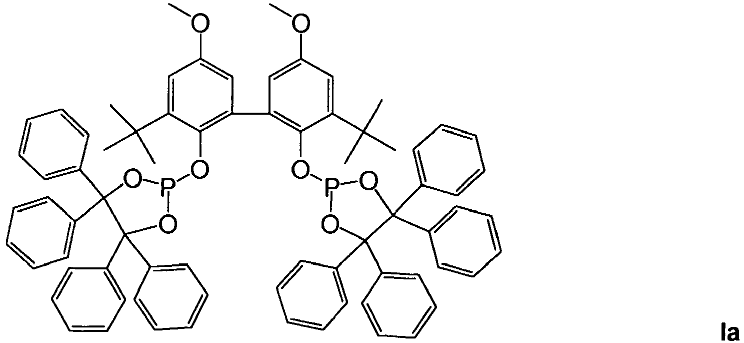

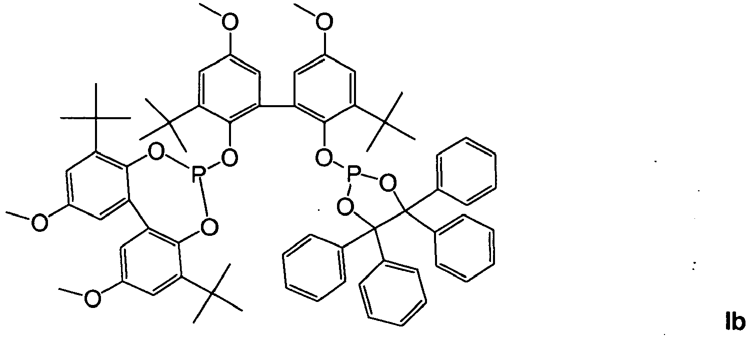

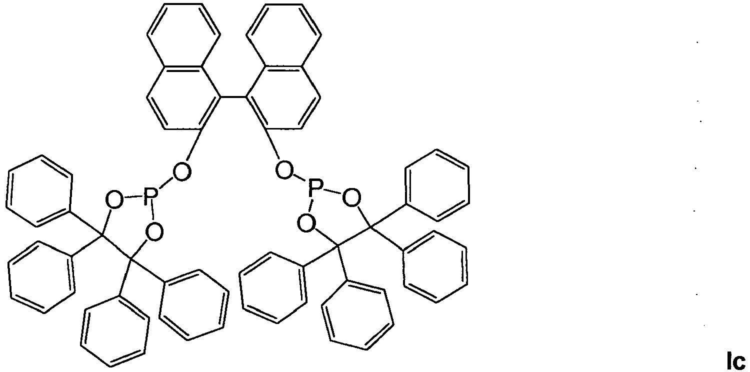

Ganz besonders bevorzugt wird ein Katalysatorsystem, bestehend aus Rhodium und dem Bisphosphit mit folgender Strukturformel I**a**, verwendet.

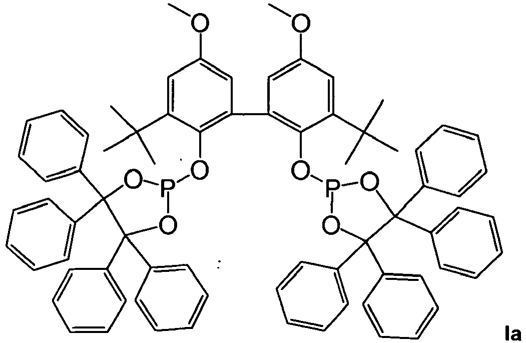

Im Katalysatorsystem liegt das molare Verhältnis von Bisphosphit zum Rhodium im Bereich von weniger als 100 : 1 bis 1 : 1, insbesondere im Bereich von 90 : 1 bis 2 : 1, ganz besonders im Bereich von 10 : 1 bis 2 : 1.

Das Rhodium kann in Form von Salzen oder Komplexen zum Einsatz kommen, z. B. als Rhodiumcarbonyle, Rhodiumnitrat, Rhodiumchlorid, Rh(CO)₂(acac) (acac = Acetylacetonat), Rhodiumacetat oder Rhodiumcarboxylate oder Rhodiumoctanoat.

Aus den Bisphosphitliganden und der Rhodiumverbindung bildet sich unter Reaktionsbedingungen die aktive Katalysatorspezies für die homogene Katalyse. Bei der Hydroformylierung bildet sich bei Kontakt des erfindungsgemäßen Bisphosphitliganden und des Katalysatormetalls mit Synthesegas vermutlich ein Carbonylhydridphosphit-Komplex als aktive Katalysatorspezies. Die Bisphosphite und gegebenenfalls weitere Liganden können in freier Form zusammen mit dem Katalysatormetall (als Salz oder Komplex) in die Reaktionsmischung gegeben werden, um die aktive Katalysatorspezies in situ zu erzeugen. Es ist weiterhin auch möglich, einen erfindungsgemäßen Phosphitmetallkomplex, der die o. g. Bisphosphitliganden und das Rhodiummetall enthält, als Vorstufe für den eigentlichen katalytisch aktiven Komplex einzusetzen. Diese Phosphitmetallkomplexe werden hergestellt, indem das Rhodium in Form einer chemischen Verbindung oder in der Oxidationsstufe 0 mit dem erfindungsgemäßen Bisphosphitliganden umgesetzt wird.

Frisches Bisphosphit kann zu jedem Zeitpunkt der Reaktion zugesetzt werden, um z. B. die Konzentration an freiem Liganden konstant zu halten.

Die Konzentration des Rhodiums im Hydroformylierungsgemisch beträgt vorzugsweise von 1 Massen-ppm bis 1000 Massen-ppm und bevorzugt 5 Massen-ppm bis 300 Massen-ppm, bezogen auf das Gesamtgewicht der Reaktionsmischung.

Die mit den entsprechenden Rhodiumkomplexen durchgeführten Hydroformylierungsreaktionen können nach bekannten Vorschriften, wie z. B. in J. FALBE, "New Syntheses with Carbon Monoxide", Springer Verlag, Berlin, Heidelberg, New York, Seite 95 ff., (1980) beschrieben, durchgeführt werden. 1-Buten wird dabei in Gegenwart des Katalysators mit einem Gemisch aus CO und H₂ (Synthesegas) zu C₅-Aldehyden umgesetzt.

Dabei enthält die C₅-Aldehydfraktion einen Anteil von größer als 90 Massen-%, insbesondere einen von größer 93 Massen-% an n-Valeraldehyd, besonders bevorzugt von größer 95 Massen-% und ganz besonders bevorzugt von größer 98 Massen-% n-Valeraldehyd.

Die Hydroformylierung wird bei Temperaturen von 40 bis 120 °C, bevorzugt bei 40 bis 110 °C, besonders bevorzugt bei 60 bis 95 °C, ganz besonders bevorzugt bei 70 bis 95 °C durchgeführt. Der Druck beträgt dabei 0,1 bis 30 MPa, insbesondere 1 MPa bis 6,4 MPa. Das Molverhältnis zwischen Wasserstoff und Kohlenmonoxid (H₂/CO) im Synthesegas beträgt vorzugsweise 10/1 bis 1/10 und besonders bevorzugt 1/1 bis 2/1.

Das Katalysatorsystem ist vorzugsweise homogen im flüssigen Hydroformylierungsgemisch, bestehend aus Edukten (Olefinen und gelöstes Synthesegas) und Produkten (Aldehyden, Alkoholen, im Prozess gebildete Nebenprodukte, insbesondere Hochsieder), gelöst. Optional können zusätzlich Lösungsmittel und/oder Stabilisatorverbindungen, wie beispielsweise sterisch gehinderte sekundäre Amine oder Promotoren verwendet werden.

Das Verfahren kann diskontinuierlich oder bevorzugt kontinuierlich durchgeführt werden. Bei einem kontinuierlichen Verfahren ist es, um einen nahezu vollständigen 1-Buten-Umsatz zu erreichen, zweckmäßig, die Umsetzung in mehreren Reaktoren durchzuführen. Beispielsweise können mehrere Rührreaktoren und/oder Blasensäulenreaktoren in Reihe geschaltet werden. Dabei kann der flüssige und/oder gasförmige Austrag eines Reaktors in den nächsten geleitet werden. Es ist auch möglich, aus einem Reaktor nicht umgesetzte Einsatz-Kohlenwasserstoffe zusammen mit Produkten und überschüssigem Synthesegas abzuziehen, daraus die C₅-Produkte auszukondensieren und das Restgas in den nächsten Reaktor einzuleiten. Die Reaktion kann auch in einer Rohrschlange mit Gaszwischeneinspeisung durchgeführt werden. Weiterhin kann die Reaktion in einer Kombination der genannten Reaktortypen durchgeführt werden.

Wenn nicht die maximale Ausbeute an C₅-Aldehyden, sondern eine hohe Raum-ZeitAusbeute angestrebt wird, kann die Hydroformylierung zumindest teilweise bei Temperaturen von über 100 °C betrieben werden. Bei diesen Temperaturen tritt während der Hydroformylierung eine teilweise Isomerisierung von 1-Buten zu den beiden 2-Butenen auf. Beispielsweise könnte(n) in einer Reaktorkaskade der erste Reaktor oder die ersten Reaktoren bei Temperaturen von über 100 °C und der oder die nachfolgenden Reaktor(en) bei Temperaturen von unter 100 °C, beispielsweise 90 °C betrieben werden

Das Hydroformylierungsprodukt kann destillativ in mindestens drei Fraktionen getrennt werden, nämlich in die Isobutenfraktion, Produktfraktion (hauptsächlich C₅-Aldehyde) und eine Hochsiederfraktion mit dem gelösten Katalysatorsystem und eine oder zwei weitere Fraktion(en), die andere nicht umgesetzte Stoffe aus dem Edukt enthält/enthalten.

Beispielsweise kann das Reaktionsgemisch des letzten Reaktors nach Abtrennung des überschüssigen Synthesegas, das in den Reaktor zurückgeführt oder nach Auskondensation darin vorhandener Stoffe ganz oder teilweise ausgeschleust werden kann, durch eine erste Destillation in ein C₄-Kohlenwasserstoffgemisch und ein Gemisch aus C₅-Produkten, Hochsiedern und Katalysatorsystem aufgetrennt wird. Die beiden Gemische werden in weiteren Destillationsschritten getrennt.

Eine andere Möglichkeit besteht darin, aus dem Reaktionsgemisch, beginnend mit dem niedrigsten siedenden Stoff oder Stoffgemisch, eine Fraktion nach der anderen abzutrennen, bis nur die Hochsieder mit dem gelösten Katalysatorsystem übrig bleiben.

Optional kann vor der destillativen Aufarbeitung ein Teil des Katalysators durch Nanofiltration abgetrennt werden, wie beispielsweise in DE 10 2005 046250 beschrieben.

Wurde beispielweise als Edukt ein C₄-Kohlenwasserstoffgemisch eingesetzt, das Isobutan, n-Butan, 1-Buten, die beiden 2-Butene und Isobuten enthielt, so werden folgende Fraktionen erhalten:
a) Isobutan (Siedepunkt -11,7 °C)
b) Isobuten (Sdp. -6,9 °C)
c) Gemisch aus n-Butan (Sdp. -0,5 °C), E-2-Buten (Sdp. 0,9 °C) und Z-2-Buten (Sdp. 3,7 °C)
d) Gemisch aus C₅-Aldehyden mit Valeraldehyd (Sdp. 103 °C), 2-Methylbutanal (Sdp. 92 - 93 °C) und 3-Methylbutanal (Sdp. 92,5 °C)
e) Hochsieder mit dem gelösten Katalysatorsystem

Das nach dem erfindungsgemäßen Verfahren abgetrennte Isobuten weist eine Reinheit von über 99 % auf. Sein Gehalt an linearen Butenen liegt dementsprechend unter 1 %. Es kann für die in der Einleitung genannten Zwecke genutzt werden.

Die C₅-Aldehydfraktion enthält geringe Mengen an Alkoholen, die durch Hydrierung der Aldehyde entstehen. Der Gehalt an Valeraldehyd in dieser Fraktion liegt bei über 95 Massen-%. Aus diesem Gemisch kann reiner Valeraldehyd abgetrennt werden. Valeraldehyd ist u. a. Zwischenprodukt für n-Pentanol. N-Pentansäure, n-Pentylamine oder n-Pentylchlorid.

Durch Aldolkondensation der C₅-Aldehydfraktion und Totalhydrierung des Aldolkondensats wird ein Decanolgemisch mit über 90 Massen-% 2-Propylheptanol gewonnen, das ein begehrtes Zwischenprodukt für die Herstellung von Weichmachern, Detergenzien und Schmiermitteln ist. Durch Aldolkondensation der C₅-Aldehydfraktion, Hydrierung der olefinischen Doppelbindung des Aldolkondensats und anschließende Oxidation der aldehydischen Gruppe kann ein Decansäuregemisch mit einem hohen Anteil an 2-Propylheptansäure erhalten werden, die beispielsweise zur Herstellung von Schmiermitteln oder Detergenzien verwendet werden können.

Die Fraktion mit den 2-Butenen kann unterschiedlich verwertet werden. Eine Möglichkeit ist die Herstellung von Oligomeren, von denen insbesondere die Dimeren und Trimeren wertvolle Zwischenprodukte für die Herstellung von Weichmachern und Alkohole darstellen. Die Oligomerisation kann dabei unter Verwendung von sauren oder Nickel enthaltenden Katalysatoren durchgeführt werden. Wenn wenig verzweigte Produkte angestrebt werden, ist eine Oligomerisation mit einem Nickel enthaltenden, heterogenen Katalysatorsystem vorteilhaft. Ein Verfahren dieser Art ist beispielsweise der OCTOL-Prozess der Evonik Oxeno GmbH.

Eine andere Verwertung der 2-Butene besteht darin, sie zu C₅-Aldehyden zu hydroformylieren. Die Hydroformylierung kann mit unterschiedlichen Katalysatoren durchgeführt werden, wobei üblicherweise ein Gemisch aus 2-Methylbutanal und n-Valeraldehyd entsteht. Wenn ein hoher Anteil von Valeraldehyd im C₅-Aldehydgemisch angestrebt wird, ist die Verwendung anderer Katalysatoren zweckmäßig. Beispielsweise wird in den Schriften DE 101 08 474, DE 101 08 475,

DE 101 08 476 und DE 102 25 282 für die Hydroformylierung von 2-Butenen ein Katalysator, bestehend aus Rhodium und einem Diphosphinliganden, der ein Xanthengerüst aufweist, eingesetzt. Unter Verwendung dieses Katalysators ist das Verhältnis zwischen Valeraldehyd und 2-Methylbutanal größer als 85:15. Hohe Selektivitäten an Valeraldehyd (größer 95 %) können bei der Verwendung eines Katalysators, bestehend aus Rhodium und sterisch anspruchsvollen aromatischen Bisphosphiten, erhalten werden, wie beispielsweise in EP 0 213 639 beschrieben.

Das so erhaltene C₅-Aldehydgemisch kann beispielsweise durch Aldolkondensation und anschließende Hydrierung des Aldolkondensats zu einem Decanolgemisch umgesetzt werden. Bei C₅-Aldehydgemischen, bei denen der Anteil an n-Valeraldehyd unter 95 % liegt, empfiehlt es sich, einen Teil des 2-Methylbutanals destillativ abzutrennen, um ein hochwertiges Decanolgemisch mit einem Gehalt an 2-Propylheptanol von über 90 % zu erhalten.

Das abgetrennte 2-Methylbutanal kann für verschiedene Zwecke eingesetzt werden, beispielsweise zur Herstellung von Isopren.

Wenn die Herstellung von Decanol aus einem C₄-Kohlenwasserstoffgemisch das Ziel ist, ist es zweckmäßig, die C₅-Aldehydgemische beider Hydroformylierungen zusammen zu aldolisieren.

### Beispiele

Der Hydroformylierungsversuch wurde in einem Parr-100ml-Autoklaven mit Druckkonstanthaltung, Gasflussmessung und Flügelrührer durchgeführt. Der Autoklav wurde unter Argonatmosphäre mit allen unten genannten Verbindungen, jedoch noch nicht mit dem zu hydroformylierenden Olefingemisch befüllt. Nach Austausch der Argonatmosphäre durch Spülen mit Synthesegas (CO/H₂ 1 : 1) wurde das Reaktionsgemisch unter Rühren (1000 U/min) und unter Synthesegasdruck auf die jeweils genannte Temperatur aufgeheizt und danach der genaue Solldruck von 2 MPa eingestellt. Anschließend wurde das zu hydroformylierende Olefingemisch zugegeben. Der Synthesegasdruck wurde während der gesamten Reaktionsdauer über einen Druckregler konstant gehalten. Die Reaktionsdauer der Hydroformylierungsversuche betrug jeweils 720 min, wobei zwischenzeitlich Proben aus dem Autoklaven für die GC-Analyse entnommen wurden. Anschließend wurde das Reaktionsgemisch auf Raumtemperatur abgekühlt, der Autoklav entspannt und mit Argon gespült.

Alle Versuche enthielten 6 g des zu hydroformylierenden Olefingemischs (Raffinat I der unten angegebenen Zusammensetzung), 50 g eutektisches Gemisch von Biphenyl und Biphenylether (Diphyl^{®}, Fa. Lanxess) sowie Bis-(2,2,6,6-tetramethyl-4-piperidyl)-sebacat (Fa. Ciba) im molaren Verhältnis von 1 : 1 zum verwendeten Liganden **Ia**. Als Präkatalysator wurde (Acetylacetonato)dicarbonylrhodium (Fa. Umicore) eingesetzt.

Es wurden Versuche bei 90 °C, 100 °C, 110 °C und 120 °C durchgeführt. Das Verhältnis von Ligand **Ia** zu Rhodium betrug in allen Fällen 5:1, die Rhodiumkonzentration 40 ppm und der Synthesegasdruck 2 MPa.

Die Analytik wurde mittels Gaschromatographie durchgeführt.

Das Raffinat I hatte jeweils die in Tabelle 1 angegebene Startzusammensetzung (alle Werte in Massen-%):

**Tabelle 1**

| Komponente | **Isobutan** | **n-Butan** | **E-2-Buten** | **1-Buten** | **Isobuten** | **Z-2-Buten** | **1,3-Butadien** | **Rest** |
|---|---|---|---|---|---|---|---|---|
| in Massen-%I | 4,09 | 10,63 | 7,69 | 26,81 | 45,72 | 4,68 | 0,18 | 0,20 |

### Beispiel: Hydroformylierung von Raffinat I bei verschiedenen Temperaturen

Die Hydroformylierung von Raffinat I obiger Zusammensetzung wurde bei 90 °C, 100 °C, 110 °C und 120 °C durchgeführt. In allen Fällen konnte nach 720 Minuten kein 1-Buten mehr nachgewiesen werden, womit quantitativer Umsatz des 1-Butens erfolgt war. Zugleich lag der jeweilige Isobutenumsatz (Bildung von 3-Methylbutanal) nach 720 Minuten bei folgenden Werten:

| | |
|---|---|
| 90 °C: | 0,58 % |
| 100 °C: | 0,67 % |
| 110 °C: | 0,80 % |
| 120 °C: | 1,17 % |

Die Hydrierung zu gesättigten Spezies spielte ebenfalls nur eine untergeordnete Rolle und lag bei weniger als einem Prozentpunkt bzw. bei Temperaturen ab 110 °C bei weniger als eineinhalb Prozentpunkten.

Bei den Versuchen bei 110 °C und 120 °C trat in gewissem Umfang gegen Reaktionsende hin auch eine isomerisierende Hydroformylierung der 2-Butene auf, so dass die Ausbeute an n-Valeraldehyd in diesen Fällen nach 720 Minuten bei 102 % bzw. 105 % bezogen auf eingesetztes 1-Buten im Raffinat I betrug.

Das aus 1-Buten (und ab 110 °C zu geringen Teilen aus 2-Butenen) nach 720 Minuten gebildete Pentanal wurde mit folgenden Linearitäten erhalten:

| | |
|---|---|
| 90 °C: | 99,1 % |
| 100 °C: | 99,0 % |
| 110 °C: | 98,9 % |
| 120 °C: | 98,4 % |

Bei den zu 100 % fehlenden Differenzwerten handelt es sich um 2-Methylbutanal.

## Patentansprüche

1. Verfahren zur Abtrennung von 1 -Buten aus C₄-haltigen Kohlenwasserstoffgemischen, enthaltend Isobuten und 1-Buten, durch Hydroformylierung, wobei das verwendete Katalysatorsystem aus einem der Übergangsmetalle der Gruppen 8 bis 10, bevorzugt Rhodium, und einem Bisphosphitliganden einer der folgenden Formeln **I-1** bis **I-3** 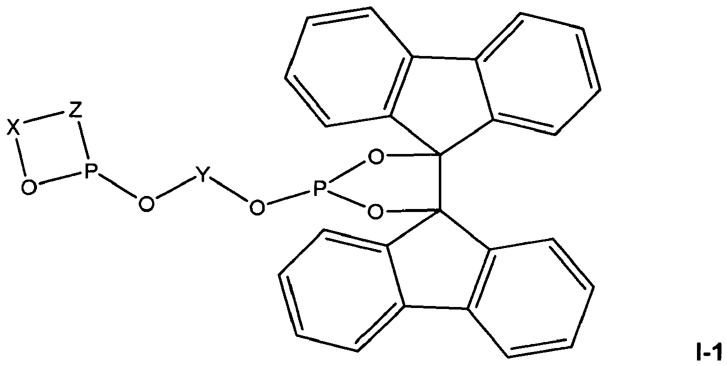 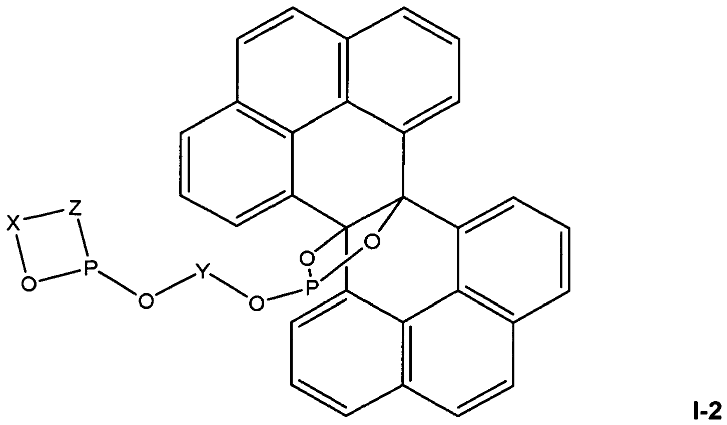 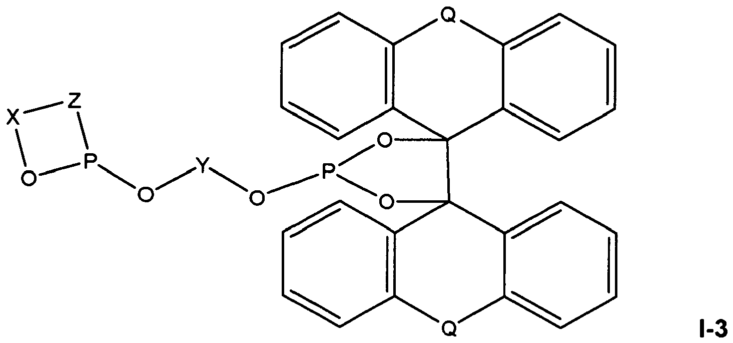 mit
X = zweiwertiger substituierter oder unsubstituierter Bisalkylen- oder Bisarylenrest, der ein oder mehrere Heteroatom(e) enthält,
Y = zweiwertiger substituierter oder unsubstituierter Bisarylen- oder Bisalkylenrest, der ein oder mehrere Heteroatom(e) enthält,
Z = Sauerstoff oder NR⁹, und R⁹ = Wasserstoff oder substituierter oder unsubstituierter Alkyl- oder Arylrest, der ein oder mehrere Heteroatom(e) enthält, Q = gleich oder verschieden CH₂, CR⁹R¹⁰, CHR⁹, O, NH oder NR⁹ sein kann, wobei R⁹ und R¹⁰ gleich oder unterschiedlich sein können, und R⁹ und R¹⁰ = Wasserstoff oder substituierter oder unsubstituierter Alkyl- oder Arylrest, der ein oder mehrere Heteroatom(e) enthält,
besteht,
wobei der Bisphosphitligand der oben angegebenen Formeln **I-1** bis **I-3** in einem Überschuss eines molaren Verhältnis von 100 : 1 bis 1 : 1 zum Übergangsmetall eingesetzt wird, und
wobei das im Einsatzgemisch vorliegende 1-Buten zu über 95 % und das im Einsatzgemisch vorhandene Isobuten zu weniger als 5 % umgesetzt wird.

2. Verfahren nach Anspruch 1, **dadurch gekennzeichnet, dass** die Konzentration des Übergangsmetalls im Hydroformylierungsgemisch von 1 Massen-ppm bis zu 1000 Massen-ppm bezogen auf das Gesamtgewicht der Reaktionsmischung beträgt.

3. Verfahren nach den Ansprüchen 1 bis 2, **dadurch gekennzeichnet, dass** das molare Verhältnis von Bisphosphit zu Rhodium im Bereich von 90 : 1 bis 1 : 1 liegt.

4. Verfahren nach den Ansprüchen 1 bis 3, **dadurch gekennzeichnet, dass** das molare Verhältnis von Bisphosphit zu Rhodium im Bereich von 10 : 1 bis 2 : 1 liegt.

5. Verfahren nach den Ansprüchen 1 bis 4, **dadurch gekennzeichnet, dass** das im Einsatzgemisch vorliegende 1-Buten zu über 99 % und das im Einsatzgemisch vorhandene Isobuten zu weniger als 5 % umgesetzt wird.

6. Verfahren nach den Ansprüchen 1 bis 5, **dadurch gekennzeichnet, dass** das 1-Buten mit einer n-/iso-Selektivität von mehr als 97 % hydroformyliert wird.

7. Verfahren nach den Ansprüchen 1 bis 6, **dadurch gekennzeichnet, dass** die Hydroformylierung in einem Druckbereich von 0,5 bis 30 MPa durchgeführt wird.

8. Verfahren nach den Ansprüchen 1 bis 7, **dadurch gekennzeichnet, dass** die Hydroformylierung in einem Druckbereich von 1,0 bis 6,4 MPa durchgeführt wird.

9. Verfahren nach den Ansprüchen 1 bis 8, **dadurch gekennzeichnet, dass** das Verfahren kontinuierlich durchgeführt wird.

10. Verfahren nach den Ansprüchen 1 bis 9, **dadurch gekennzeichnet, dass** das C₄-haltige Kohlenwasserstoffgemisch 2-Butene enthält.

11. Verfahren nach den Ansprüchen 1 bis 10, **dadurch gekennzeichnet, dass** das Reaktionsgemisch in C₅-Aldehyde, Isobuten, eine Hochsiederfraktion mit dem gelösten Katalysatorsystem und weitere C₄-Kohlenwasserstoffe aufgetrennt wird.

12. Verfahren nach Anspruch 11, **dadurch gekennzeichnet, dass** die weiteren C₄-Kohlenwasserstoffe einer isomerisierenden Hydroformylierung zu Valeraldehyd unterworfen werden.

13. Verfahren nach Anspruch 12, **dadurch gekennzeichnet, dass** die aus Selektivhydroformylierung und isomerisierender Hydroformylierung gewonnenen C₅-Aldehyde vereinigt werden.

14. Verfahren nach Ansprüchen 11 oder 13, **dadurch gekennzeichnet, dass** die abgetrennten C₅-Aldehyde einer Aldolkondensation, Dehydratisierung und anschließender Hydrierung zu 2-Propylheptanol unterworfen werden.

## Claims

1. Process for removing 1-butene from C₄-containing hydrocarbon mixtures comprising isobutene and 1-butene by hydroformylation, the catalyst system used consisting of one or the transition metals of groups 8 to 10, preferably rhodium, and a bisphosphite ligand of one of the following formulae **I-1** to **I-3** 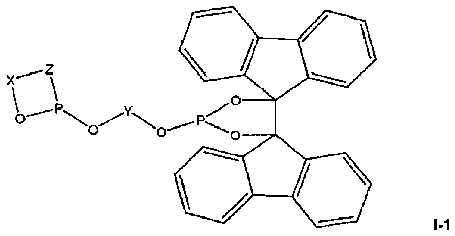 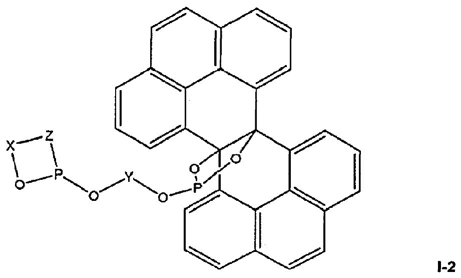 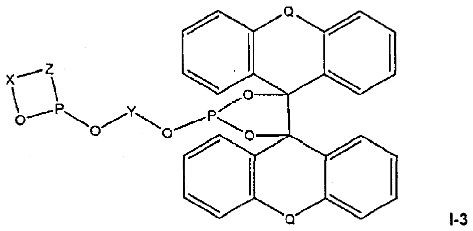 where
X = divalent substituted or unsubstituted bisalkylene or bisarylene radical which contains one or more heteroatom (s),
Y = divalent substituted or unsubstituted bisarylene or bisalkylene radical which contains one or more heteroatom (s),
Z = oxygen or NR⁹, and R⁹ = hydrogen or substituted or unsubstituted alkyl or aryl radical which contains one or more heteroatom(s), Q may be the same or different and may be CH₂, CR⁹R¹⁰, CHR⁹, O, NH or NR⁹, where R⁹ and R¹⁰ may be the same or different, and R⁹ and R¹⁰ = hydrogen or substituted or unsubstituted alkyl or aryl radical containing one or more heteroatom (s),
the bisphosphite ligand of the above-specified formulae **I-1** to **I-3** being used in an excess of a molar ratio of 100 : 1 to 1 : 1 relative to the transition metal, and
the 1-butene present in the starting mixture being converted to an extent of more than 95% and the isobutene present in the starting mixture to an extent of less than 5%.

2. Process according to claim 1, **characterized in that** the concentration of the transition metal in the hydroformylation mixture is 1 ppm by mass up to 1000 ppm by mass based on the total weight of the reaction mixture.

3. Process according to either of claims 1 and 2, **characterized in that** the molar ratio of bisphosphite to rhodium is in the range from 90 : 1 to 1 : 1.

4. Process according to any one of claims 1 to 3, **characterized in that** the molar ratio of bisphosphite to rhodium is in the range from 10 : 1 to 2 : 1.

5. Process according to any one of claims 1 to 4, **characterized in that** the 1-butene present in the starting mixture is converted to an extent of more than 99% and the isobutene present in the starting mixture to an extent of less than 5%.

6. Process according to any one of claims 1 to 5, **characterized in that** the 1-butene is hydroformylated with an n-/iso selectivity of more than 97%.

7. Process according to any one of claims 1 to 6, **characterized in that** the hydroformylation is carried out within a pressure range from 0.5 to 30 MPa.

8. Process according to any one of claims 1 to 7, **characterized in that** the hydroformylation is carried out within a pressure range from 1.0 to 6.4 MPa.

9. Process according to any one of claims 1 to 8, **characterized in that** the process is performed continuously.

10. Process according to any one of claims 1 to 9, **characterized in that** the C₄-containing hydrocarbon mixture comprises 2-butenes.

11. Process according to any one of claims 1 to 10, **characterized in that** the reaction mixture is separated into C₅ aldehydes, isobutene, a high boiler fraction comprising the dissolved catalyst system and further C₄ hydrocarbons.

12. Process according to claim 11, **characterized in that** the further C₄ hydrocarbons are subjected to an isomerizing hydroformylation to give valeraldehyde.

13. Process according to claim 12, **characterized in that** the C₅ aldehydes obtained from selective hydroformylation and isomerizing hydroformylation are combined.

14. Process according to claim 11 or 13, **characterized in that** the C₅ aldehydes removed are subjected to an aldol condensation, dehydration and subsequent hydrogenation to 2-propylheptanol.

## Revendications

1. Procédé de séparation de 1-butène de mélanges d'hydrocarbures contenant des composés en C₄, contenant de l'isobutène et du 1-butène, par hydroformylation, le système catalytique utilisé étant constitué d'un des métaux de transition des groupes 8 à 10, de préférence de rhodium, et d'un ligand bisphosphite ayant une des formules I-1 à I-3 suivantes 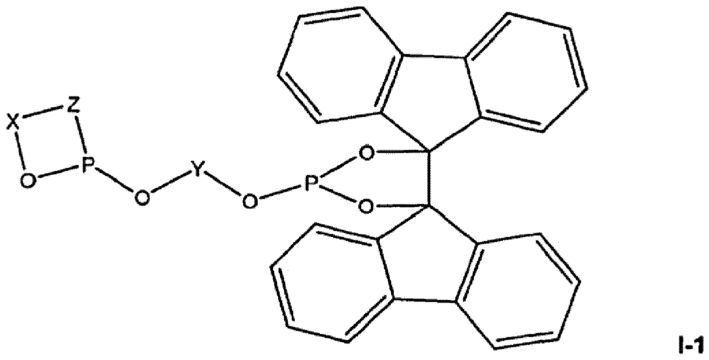 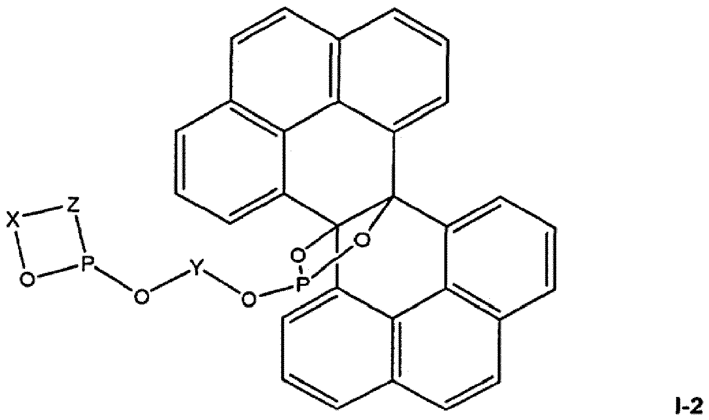 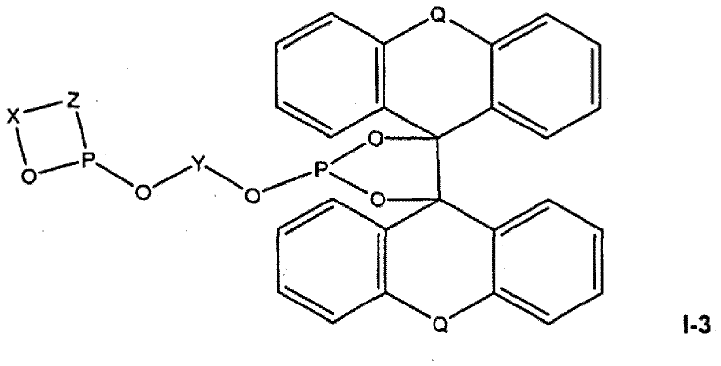 avec
X = radical bisalkylène ou bisarylène bivalent substitué ou non substitué, qui contient un ou plusieurs hétéroatomes,
Y = radical bisarylène ou bisalkylène bivalent substitué ou non substitué, qui contient un ou plusieurs hétéroatomes,
Z = oxygène ou NR⁹, et R⁹ = hydrogène ou radical alkyle ou aryle substitué ou non substitué, qui contient un ou plusieurs hétéroatomes,
Q = peuvent être identiques ou différents et représentent CH₂, CR⁹R¹⁰, CHR⁹, 0, NH ou NR⁹, R⁹ et R¹⁰ pouvant être identiques ou différents, et R⁹ et R¹⁰ = hydrogène ou radical alkyle ou aryle substitué ou non substitué, qui contient un ou plusieurs hétéroatomes,
le ligand bisphosphite des formules I-1 à I-3 indiquées ci-dessus étant utilisé en un excès d'un rapport molaire de 100:1 à 1:1 par rapport au métal de transition et
le 1-butène présent dans le mélange initial étant transformé à plus de 95 % et l'isobutène présent dans le mélange initial à moins de 5 %.

2. Procédé selon la revendication 1, **caractérisé en ce que** la concentration du métal de transition dans le mélange d'hydroformylation est de 1 ppm en masse à 1 000 ppm en masse par rapport au poids total du mélange réactionnel.

3. Procédé selon les revendications 1 à 2, **caractérisé en ce que** le rapport molaire entre le bisphosphite et le rhodium se situe dans la plage allant de 90:1 à 1:1.

4. Procédé selon les revendications 1 à 3, **caractérisé en ce que** le rapport molaire entre le bisphosphite et le rhodium se situe dans la plage allant de 10:1 à 2:1.

5. Procédé selon les revendications 1 à 4, **caractérisé en ce que** le 1-butène présent dans le mélange initial est transformé à plus de 99 % et l'isobutène présent dans le mélange initial à moins de 5 %.

6. Procédé selon les revendications 1 à 5, **caractérisé en ce que** le 1-butène est hydroformylé avec une sélectivité n/iso supérieure à 97 %.

7. Procédé selon les revendications 1 à 6, **caractérisé en ce que** l'hydroformylation est réalisée dans une plage de pression de 0,5 à 30 MPa.

8. Procédé selon les revendications 1 à 7, **caractérisé en ce que** l'hydroformylation est réalisée dans une plage de pression de 1,0 à 6,4 MPa.

9. Procédé selon les revendications 1 à 8, **caractérisé en ce que** le procédé est réalisé en continu.

10. Procédé selon les revendications 1 à 9, **caractérisé en ce que** le mélange d'hydrocarbures contenant des composés en C₄ contient des 2-butènes.

11. Procédé selon les revendications 1 à 10, **caractérisé en ce que** le mélange réactionnel est séparé en aldéhydes en C₅, isobutène, une fraction de composés de point d'ébullition élevé contenant le système catalytique dissous et d'autres hydrocarbures en C₄.

12. Procédé selon la revendication 11, **caractérisé en ce que** les autres hydrocarbures en C₄ sont soumis à une hydroformylation isomérisante pour former du valéraldéhyde.

13. Procédé selon la revendication 12, **caractérisé en ce que** les aldéhydes en C₅ obtenus par l'hydroformylation sélective et l'hydroformylation isomérisante sont réunis.

14. Procédé selon la revendication 11 ou 13, **caractérisé en ce que** les aldéhydes en C₅ séparés sont soumis à une condensation d'aldol, une déshydratation et une hydrogénation ultérieure pour former du 2-propylheptanol.
